# FASCICULE DE BREVET EUROPEEN

(11) **EP 1 248 789 B1**
(45) Date de publication et mention de la délivrance du brevet: **30.03.2005**
(21) Numéro de dépôt: 01907606.6
(22) Date de dépôt: 17.01.2001
(51) Int. Cl.: C07F 7/08

(54) **COMPOSES ORGANOSILICIES, LEUR PROCEDE DE PREPARATION ET LEURS UTILISATIONS**
ORGANOSILICIUM-VERBINDUNGEN, IHRE HERSTELLUNG UND IHRE VERWENDUNG
ORGANOSILICON COMPOUNDS, PREPARATION METHOD AND USES THEREOF

(30) Priorité: 20.01.2000 FR 0000695
(43) Date de publication de la demande: 16.10.2002
(73) Titulaire: Centre National de la Recherche Scientifique (CNRS), 75016 Paris (FR)
(72) Inventeur: BENNETAU, Bernard, F-33400 Talence (FR); BOUSBAA, Jamal, F-33405 Talence Cedex (FR); CHOPLIN, Franck, F-72320 Valennes (FR)
(74) Mandataire: Goulard, Sophie
(86) Numéro de dépôt international: PCT/FR2001/000139
(87) Numéro de publication internationale: WO 2001/053303

(56) Documents cités:
- EP-A- 0 482 613
- EP-A- 0 552 637
- EP-A- 0 629 673
- PATENT ABSTRACTS OF JAPAN vol. 1999, no. 09, 30 juillet 1999 (1999-07-30) & JP 11 092251 A (MATSUSHITA ELECTRIC IND CO LTD), 6 avril 1999 (1999-04-06)
- PATENT ABSTRACTS OF JAPAN vol. 1995, no. 05, 30 juin 1995 (1995-06-30) & JP 07 051355 A (KURARAY CO LTD), 28 février 1995 (1995-02-28)

## Description

La présente invention se rapporte à des composés organosiliciés, à leur procédé de préparation et à leur utilisation pour déposer une monocouche autoassemblée de ces composés à la surface d'un support solide. La présente invention se rapporte également aux supports solides ainsi modifiés et à leur procédé d'obtention, ainsi qu'à leur utilisation pour la synthèse ou l'immobilisation de biomolécules.

Une monocouche autoassemblée organisée (aussi appelée SAM : « Self-Assembled Monolayer ») est définie comme un assemblage de molécules dans lesquel les molécules sont organisées, organisation due à des interactions entre les chaînes des molécules, donnant lieu à un film anisotrope stable, monomoléculaire et ordonné (A. ULMAN, *Chem*. *Rev.,* 1996, *96*, 1533-1554). Ces monocouches autoassemblées, que l'on peut obtenir de façon reproductible (J.B. BROZSKA *et al., Langmuir,* 1994, *10*, 4367-4373), ont la particularité de former un film dense, homogène et résistant aux traitements chimiques (acide ou basique).

La formation de SAM sur des supports solides, en utilisant l'octadécyltrichlorosilane par exemple, permet la préparation de surfaces organiques homogènes et de paramètres bien définis à la fois chimiquement et structurellement. Ces surfaces peuvent servir de modèles à deux dimensions pour des études fondamentales, notamment en ce qui concerne les phénomènes d'autoassémblage et la chimie des interfaces (A. ULMAN, *ibid*).

Divers composés organosiliciés ont été utilisés comme agents de couplage pour la fonctionnalisation de supports solides (L.A. CHRISEY *et al., Nucleic Acids Research,* 1996, *24*, 15, 3031-3039, U. MASKOS *et al., Nucleic Acids Research,* 1992, *20*, 7, 1679-1684) dans le but d'immobiliser ou de synthétiser *in situ* des oligonucléotides. Toutefois, les agents de couplage organosiliciés utilisés dans ces travaux forment des films inhomogènes et très peu résistants aux traitements chimiques ultérieurs de synthèse ou d'immobilisation d'oligonucléotides. De plus, la formation des films avec ces agents de couplage n'est pas reproductible.

Certains composés organosiliciés fluorés ont par ailleurs déjà été utilisés pour la fabrication de films fluorocarbonés afin d'améliorer la performance des appareils électriques nécessitant la présence d'un revêtement résistant à la chaleur, aux intempéries et à l'usure (EP-A-0 482 613 et EP-A-0 629 673) ou pour la réalisation de films hydrofuges et oléofuges à la surface de marbre artificiel (JP 11092251). D'autres composés organosiliciés sont également utilisés pour réaliser la synthèse de polymères conjugués de type polyacétylène sur support solide (EP-A-0 552 637).

Les Inventeurs se sont donc donnés pour but de pallier les inconvénients de l'art antérieur et de pourvoir à des agents de couplage qui permettent d'obtenir de véritables SAM à la surface de supports solides, à savoir des films monocouche stables dans lesquels les molécules soient autoassemblées et organisées. Les Inventeurs se sont également donné pour but de pourvoir à des agents de couplage dont le greffage sur des supports solides soit reproductible, tout en rendant possible des synthèses ou des immobilisations de biomolécules à la surface de la monocouche formée sur le support.

La présente invention a pour objet des composés organosiliciés de formule (I) 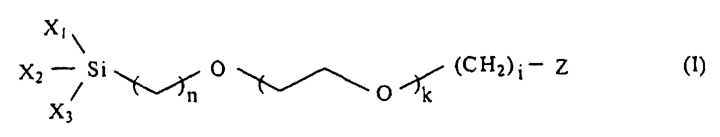 dans laquelle :
. n est compris entre 15 et 35, de préférence entre 20 et 25,
. k est compris entre 0 et 100, de préférence entre 0 et 5,
. i est un nombre entier supérieur ou égal à 0, de préférence égal à 0 ou à 1,
. X₁, X₂ et X₃, qui peuvent être identiques ou différents entre eux, sont sélectionnés dans le groupe constitué par les alkyles saturés en C₁ à C₆, linéaires ou ramifiés, et les groupements hydrolysables, au moins l'un de X₁, X₂ ou X₃ représentant un groupement hydrolysable, et
. si k = 0 et i = 0, alors Z représente un groupement R₁,
. si k = 0 et i ≥ 1, alors Z représente un groupement -OR₁, -OCOR₁, -NR₁R₂, -COOR₁, -CONR₁R₂, -SR₁ ou un atome d'halogène,
. si k ≥ 1 et i = 0, alors Z représente un groupement -R₁, -COR₁, -COOR₁, -CONR₁R₂, -CF₃ ou -(CF₂)ⱼCF₃, j étant compris entre 1 et 10,
. si k ≥ 1 et i ≥ 1, alors Z représente un groupement -OR₁, -OCOR₁, -NR₁R₂, -COOR₁, -CONR₁R₂, -SR₁, -CF₃, -(CF₂)ⱼCF₃, j étant tel que défini ci-dessus, ou un atome d'halogène,
. R₁ et R₂, qui peuvent être identiques ou différents, représentent un atome d'hydrogène, une chaîne hydrocarbonée éventuellement substituée, saturée ou insaturée et linéaire ou ramifiée, comprenant de 1 à 24 atomes de carbone, ou un groupement aromatique, à la condition que, lorsque k = i = 0 et n = 15, R₁ est différent du groupe -CH₂CF₃ et, lorsque k = i = 0 et n = 19, R₁ est différent du groupe -(CH₂)₆-C≡C-C≡CH.

Lorsque Z représente un groupement -OR₁, -OCOR₁ ou -COOR₁, indifféremment des valeurs de i, et lorsque k ≥ 1, alors il est bien entendu que Z peut représenter tout groupement résultant de la protection d'une fonction hydroxyle ou acide carboxylique, tels que les groupements protecteurs décrits dans *Protective groups in organic synthesis* (T.W. GREENE *et al*., 2^{nd} edition, Wiley Interscience), par exemple un groupement protecteur cyclique.

Au sens de la présente invention, on entend par « aromatique » tout groupement qui possède un ou plusieurs noyaux aryles, par exemple un noyau phényle. On entend par « groupe hydrolysable» tout groupe capable de réagir avec un acide en milieu aqueux de façon à donner les composés X₁H, X₂H ou X₃H.

De préférence, ledit groupement hydrolysable est sélectionné dans le groupe constitué par les atomes d'halogène, le groupement -N(CH₃)₂ et les groupements -OR, R étant un groupe alkyle saturé en C₁ à C₆, linéaire ou ramifié.

En ce qui concerne les groupements Z et les groupements hydrolysables, des atomes d'halogène convenables sont aussi bien le fluor que le chlore, le brome ou l'iode.

Les composés organosiliciés selon la présente invention présentent avantageusement des fonctionnalités très variées, eu égard à la diversité des groupements Z terminaux utilisables, ces groupements Z pouvant être modifiés et fonctionnalisés à volonté selon les réactions de chimie organique bien connues de l'Homme du métier.

Selon un mode de réalisation avantageux, un composé de formule (I) est tel que X₁, X₂ et X₃ représentent des atomes de chlore, n est égal à 22, i est égal à 0, k est égal à 1 ou à 3 et Z représente un groupement -COCH₃.

Selon un autre mode de réalisation avantageux, un composé de formule (I) est tel que X₁, X₂ et X₃ représentent des atomes de chlore, n est égal à 22, i est égal à 1, k est égal à 2 et Z représente un groupement -COOCH₃.

De manière surprenante, les produits sélectionnés permettent effectivement d'obtenir de véritables SAM à la surface de supports solides, à savoir des films monocouche stables dans lesquels les molécules soient autoassemblées et organisées.

La présente invention a également pour objet un procédé de préparation des composés de formule (I) décrite ci-avant dans laquelle i est différent de 1, procédé qui comprend les étapes suivantes :
a) préparation d'un précurseur insaturé de formule (III) : 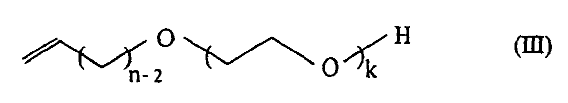 par réaction d'un diol de formule HO-(CH₂-CH₂-O)ₖ-H avec un composé insaturé de formule (II) : 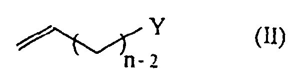 dans lesquelles Y représente un groupe nucléofuge et n et k sont tels que définis ci-avant en rapport avec la formule (I) ;
b) obtention, par fonctionnalisation de l'extrémité hydroxyle du composé de formule (III), d'un précurseur insaturé de formule (IV) : 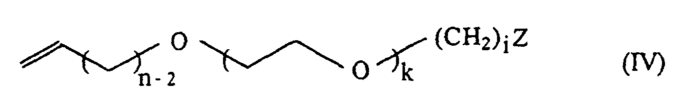 dans laquelle Z et i sont tels que définis ci-avant en rapport avec la formule (I) ;
c) obtention, par hydrosilylation du précurseur insaturé de formule (IV) à l'aide d'un hydrogénosilane de formule HSiX₁X₂X₃, d'un composé silicié de formule (I) : 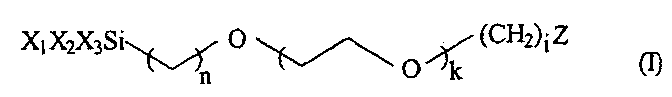 dans laquelle au moins l'un de X₁, X₂ et X₃ représente un atome d'halogène ; et
d) éventuellement, obtention d'un autre composé de formule (I) par substitution d'un ou de plusieurs des groupements X₁, X₂ et X₃ du composé obtenu à l'étape c) à l'aide de groupements X₁, X₂ et/ou X₃ tels que définis en rapport avec le composé de formule (I) selon la présente invention.

L'étape b) de fonctionnalisation de l'extrémité hydroxyle du composé de formule (III) peut par exemple être réalisée; lorsque i est égal à 0, par une réaction d'estérification à l'aide de chlorure d'alcoyle quand Z représente un groupement -COR₁, R₁ étant tel que défini en rapport avec le composé de formule (I) selon l'invention.

La présente invention a également pour objet un procédé de préparation des composés de formule (I) selon la présente invention tels que décrits ci-avant, dans laquelle i est égal à 1, procédé qui comprend les étapes suivantes :
a) préparation d'un précurseur insaturé de formule (III') : 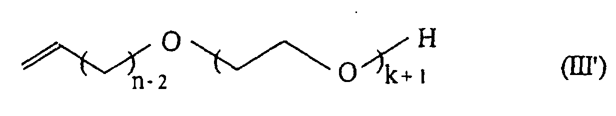 par réaction d'un composé insaturé de formule (II) telle que définie ci-dessus avec un diol de formule HO-(CH₂-CH₂-O)ₖ₊₁-H, n et k étant tels que définis ci-avant en rapport avec le composé de formule (I) selon la présente invention ;
b) obtention, par oxydation de l'extrémité hydroxyle du composé (III'), d'un précurseur insaturé de formule (IV') : 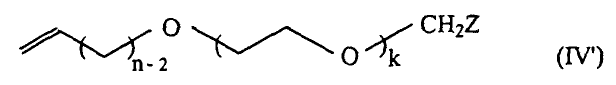 dans laquelle Z représente une fonction acide carboxylique ;
c) éventuellement, fonctionnalisation de l'extrémité acide carboxylique du composé de formule (IV') à l'aide d'un autre groupement Z tel que défini en rapport avec la formule (I) des composés selon la présente invention ;
d) obtention, par hydrosilylation du précurseur insaturé de formule (IV') à l'aide d'un hydrogénosilane de formule HSiX₁X₂X₃, d'un composé silicié de formule (I) : 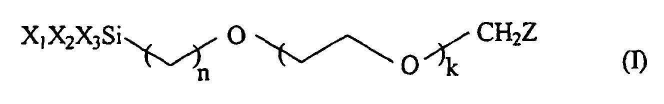 dans laquelle au moins l'un de X₁, X₂ et X₃ représente un atome d'halogène ; et
e) éventuellement, obtention d'un autre composé de formule (I) par substitution d'un ou de plusieurs des groupements X₁, X₂ et X₃ du composé obtenu à l'étape d) à l'aide de groupements X₁, X₂ et/ou X₃ tels que définis en rapport avec le composé de formule (I) selon la présente invention.

Dans les procédés décrits ci-dessus pour la préparation des composés de formule (I) selon l'invention, quelle que soit la valeur de i, l'étape a) est avantageusement réalisée dans un solvant polaire, par exemple l'eau ou le tétrahydrofurane, en milieu basique et à la température de reflux du solvant ; en tant que groupe nucléofuge Y présent dans le composé de formule (II), on peut par exemple utiliser un atome d'halogène ou un groupe tosyle ; par ailleurs, l'étape d'hydrosilylation du précurseur insaturé peut être réalisée en présence de trichlorosilane.

Les composés organosiliciés de formule (I) selon la présente invention peuvent par exemple être utilisés dans des procédés sol-gel, c'est-à-dire être hydrolysés puis réticulés de façon à obtenir de nouveaux matériaux, ou encore servir de comonomères dans des synthèses de nouveaux polymères dans le but de modifier les propriétés chimiques et mécaniques de ces polymères de par les fonctionnalités introduites, par exemple sous forme de chaînes pendantes. Ils peuvent également être utilisés pour former une monocouche autoassemblée organisée à la surface d'un support solide.

Ainsi, la présente invention a également pour objet l'utilisation d'un composé organosilicié de formule générale (I') : 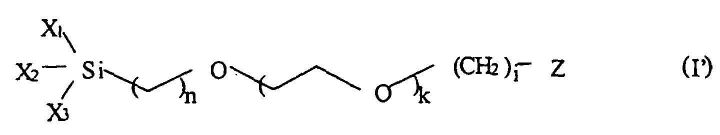 dans laquelle :
. n est compris entre 15 et 35,
. k est compris entre 0 et 100,
. i est un nombre entier supérieur ou égal à 0,
. X₁, X₂ et X₃, qui peuvent être identiques ou différents entre eux, sont sélectionnés dans le groupe constitué par les alkyles saturés en C₁ à C₆, linéaires ou ramifiés, et les groupements hydrolysables, au moins l'un de X₁, X₂ ou X₃ représentant un groupement hydrolysable, et
. si k = 0 et i = 0, alors Z représente un groupement R₁,
. si k = 0 et i ≥ 1, alors Z représente un groupement -OR₁, -OCOR₁, -NR₁R₂, -COOR₁, -CONR₁R₂, -SR₁ ou un atome d'halogène,
. si k ≥ 1 et i = 0, alors Z représente un groupement -R₁, -COR₁, -COOR₁, -CONR₁R₂, -CF₃ ou -(CF₂)ⱼCF₃, j étant compris entre 1 et 10,
. si k ≥ 1 et i ≥ 1, alors Z représente un groupement -OR₁, -OCOR₁, -NR₁R₂, -COOR₁, -CONR₁R₂, -SR₁, -CF₃, -(CF₂)ⱼCF₃, j étant tel que défini ci-dessus, ou un atome d'halogène,
. R₁ et R₂, qui peuvent être identiques ou différents, représentent un atome d'hydrogène, une chaîne hydrocarbonée éventuellement substituée, saturée ou insaturée et linéaire ou ramifiée, comprenant de 1 à 24 atomes de carbone, ou un groupement aromatique,
pour former, à la surface d'un support solide, une monocouche autoassemblée organisée.

L'utilisation des composés organosiliciés de formule générale (I') permet avantageusement de modifier la surface de supports solides par une monocouche dense et organisée, qui répond à la définition des SAM donnée précédemment. La monocouche ainsi formée sur la surface présente une grande résistance vis-à-vis des traitements chimiques (acides ou basiques). La robustesse et l'homogénéité de la monocouche formée à la surface du support par les agents organosiliciés selon la présente invention permettent par exemple de traiter des supports contre la corrosion.

Les composés greffés sur le support donnent lieu à des liaisons covalentes fortes, de type siloxanique, avec la surface et développent une forte cohésion entre leurs chaînes alkyles, résultat d'un autoassemblage des molécules qui protège les liaisons siloxaniques. En outre, le greffage est reproductible et les groupements Z des composés greffés présentent une grande réactivité chimique.

La présente invention a également pour objet un support solide dont la surface est modifiée par une monocouche autoassemblée organisée, caractérisé en ce que ladite monocouche comprend un réseau d'au moins un composé organosilicié de formule générale (I') telle que définie ci-avant.

Au sens de la présente invention, on entend par « réseau » un assemblage de molécules dans lequel les molécules sont organisées et dans lequel les chaînes des molécules interagissent entre elles par des liaisons covalentes ou non (forces de Van der Waals par exemple).

Il est bien entendu que ladite monocouche, outre des composés organosiliciés de formule générale (I') selon la présente invention, peut également comprendre tout autre type de composé capable d'être greffé sur le support solide (obtention d'une monocouche dite « mixte »), ce qui permet de diminuer la densité des composés de formule (I') sur le support, lorsqu'un tel effet est recherché.

Des supports solides convenables sont ceux dont la surface est hydratée. De préférence, ledit support solide est tel que sa surface présente, avant d'être modifiée, des groupements hydroxyles. Il est avantageusement sélectionné dans le groupe constitué par les verres, les céramiques (de préférence de type oxyde), les métaux (par exemple l'aluminium ou l'or) et les métalloïdes (tel que le silicium oxydé).

La présente invention a également pour objet un procédé d'obtention d'un support solide tel que défini ci-dessus, caractérisé en ce qu'il comprend les étapes suivantes :
a) élimination des contaminants du support solide et hydratation et/ou hydroxylation de sa surface,
b) introduction, sous atmosphère inerte, d'au moins un composé organosilicié de formule générale (I') telle que définie ci-avant dans un mélange d'au moins deux solvants comprenant au moins un solvant hydrocarboné non polaire,
c) silanisation du support obtenu à l'étape a) par immersion dans la solution préparée à l'étape b), et
d) rinçage du support modifié obtenu à l'étape c) à l'aide d'un solvant, de préférence polaire.

Par « contaminants » du support solide, on entend tout composé tel que de la graisse, des poussières ou autres, présent à la surface du support et qui ne fait pas partie de la structure chimique du support lui-même.

Le procédé selon l'invention permet avantageusement de modifier chimiquement les propriétés d'une surface minérale, et ce en fonction des groupes Z introduits par les composés organosiliciés greffés sur la surface.

De façon particulièrement avantageuse, selon la nature du support solide, l'étape a) est effectuée à l'aide d'un ou de plusieurs solvants et/ou oxydants et/ou hydroxylants (par exemple un mélange sulfochromique), d'un détergent (par exemple du Hellmanex®), d'un traitement photochimique à l'ozone ou tout autre traitement approprié.

L'étape b) peut avantageusement être réalisée dans un mélange d'au moins un solvant hydrocarboné non polaire et d'au moins un solvant polaire. Dans ce cas, les proportions volumiques du solvant non polaire par rapport au solvant polaire sont de préférence comprises entre 70/30 et 95/5. A titre d'exemples et de façon non limitative, un solvant hydrocarboné non polaire utilisable est le cyclohexane et un solvant polaire utilisable est le chloroforme.

La concentration du composé organosilicié dans le mélange de solvants, à l'étape b) du procédé selon la présente invention, est avantageusement comprise entre 1.10⁻⁵ et 1.10⁻² mole/litre.

L'étape c) de silanisation du support peut être effectuée pendant un temps compris entre 1 minute et 3 jours et à une température comprise entre -10°C et 120°C selon les solvants utilisés.

Les supports solides dont la surface est modifiée par une monocouche autoassemblée organisée selon la présente invention peuvent avantageusement être utilisés, en fonction de la nature du groupe terminal Z, en tant que supports pour la synthèse ou l'immobilisation de biomolécules, par exemple des oligonucléotides ou des protéines.

Ainsi, la présente invention a également pour objet l'utilisation d'un support solide tel que décrit ci-avant pour la synthèse ou l'immobilisation de biomolécules par liaison covalente.

La présente invention a plus particulièrement pour objet un procédé de synthèse de biomolécules sur un support solide tel que décrit ci-avant, caractérisé en ce que lesdites biomolécules sont constituées d'un enchaînement d'unités répétitives et en ce que ledit procédé comprend des étapes successives de greffage desdites unités répétitives, la première unité répétitive greffée portant une fonction réactive vis-à-vis des groupements Z des composés organosiliciés présents sur le support solide.

La présente invention a, en outre, pour objet un procédé d'immobilisation de biomolécules sur un support solide tel que décrit ci-avant, caractérisé en ce qu'il comprend une étape de greffage desdites biomolécules, qui portent des fonctions réactives vis-à-vis des groupements Z des composés organosiliciés, sur ledit support solide.

Avant de mettre en oeuvre les procédés de synthèse ou d'immobilisation de biomolécules décrits ci-dessus, et dans le cas où la fonction terminale Z des composés organosiliciés est par exemple un groupement -OCOR₁ (dans lequel R₁ est tel que défini en rapport avec la formule (I') des composés selon la présente invention) ou un groupement -COOR₁ (dans lequel R₁ est tel que défini en rapport avec la formule (I') des composés selon la présente invention, mais est différent d'un atome d'hydrogène), la fonction alcool ou acide carboxylique correspondante peut être préalablement déprotégée, si nécessaire, par un traitement chimique approprié, tel que de la potasse 0,5 M dans un mélange eau/éthanol.

Outre les applications qui viennent d'être évoquées ci-dessus, les supports solides selon la présente invention peuvent également être utilisés, à titre d'exemples et de façon non limitative, pour greffer des catalyseurs sur des supports minéraux, ou dans le domaine de la chimie combinatoire pour réaliser des synthèses chimiques variées sur supports solides. Ils peuvent également subir des modifications chimiques ultérieures : par exemple, un traitement avec des amines d'un support solide sur lequel sont greffés des composés organosiliciés bromés selon la présente invention permet d'obtenir une surface aux propriétés biocides.

Outre les dispositions qui précèdent, l'invention comprend encore d'autres dispositions qui ressortiront de la description qui va suivre, qui se réfère à des exemples de synthèse de composés organosiliciés selon la présente invention et de modification de supports solides par une monocouche autoassemblée organisée de ces composés organosiliciés, ainsi qu'aux dessins annexés, dans lesquels :
- les figures 1, 2 et 3 illustrent la synthèse de précurseurs insaturés des composés organosiliciés selon la présente invention,
- la figure 4 illustre la silylation de ces précurseurs insaturés,
- la figure 5 représente des spectres infra-rouge réalisés après trois expériences de greffage du composé organosilicié 14 sur des substrats Au/Si/SiO₂, et
- la figure 6a représente la densité, analysée par spectroscopie Raman, de la surface du substrat Au/Si/SiO₂ sur lequel sont greffés les composés organosiliciés 14; les figures 6b et 6c représentent les spectres Raman pris en deux points différents de cette surface.
   Il doit être bien entendu, toutefois, que ces exemples sont donnés uniquement à titre d'illustration de l'objet de l'invention, dont ils ne constituent en aucune manière une limitation.

### EXEMPLE 1 : Synthèse de composés organosiliciés de formule (I).

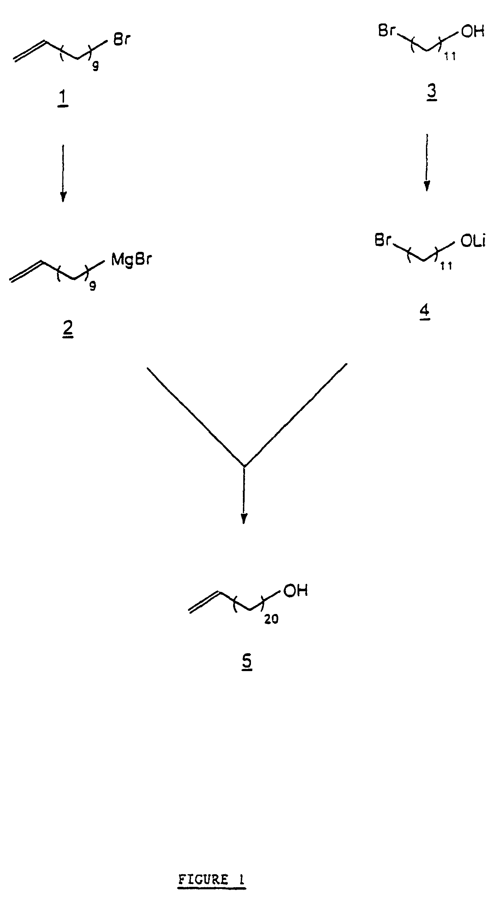

### 1) Synthèse d'un alcool insaturé (figure 1).

### • Préparation du magnésien 2

Dans un ballon tricol de 500 ml, sous atmosphère inerte, est introduit du magnésium (1,8 g; 70 mmol). Le dérivé bromé insaturé 1 (16,3 g; 70 mmol), préalablement mis en solution dans 70 ml de THF (tétrahydrofurane) anhydre, est ajouté goutte à goutte. Quelques gouttes de dibromoéthane peuvent être nécessaires pour activer le magnésium. Le mélange réactionnel est porté au reflux pendant 1 h 30 afin d'obtenir le magnésien 2, qui sera utilisé immédiatement.

### • Préparation de l'alcoolate de lithium 4

Dans un ballon tricol sec de 250 ml, sous atmosphère inerte, le bromo-alcool 3 (17,7 g; 70 mmol; 1 éq.) est mis en solution dans 70 ml de THF anhydre. La solution est refroidie à -78°C puis du méthyllithium (50 ml; 80 mmol; 1,1 éq.) est ajouté goutte à goutte. On obtient l'alcoolate de lithium 4.

### • Préparation de l'alcool insaturé 5

Le magnésien 2 est refroidi à -78°C, puis de l'iodure de cuivre (1,1 g; 3,5 mmol; 0,05 éq.) est ajouté. La solution est agitée pendant 25 minutes à -78°C, puis réchauffée à température ambiante jusqu'à obtenir une couleur pourpre. La solution est alors immédiatement refroidie à -78°C et l'alcoolate de lithium 4 est introduit à l'aide d'une canule sous atmosphère d'argon. La solution est agitée pendant 1 h à -78°C puis pendant 18 h à température ambiante. L'excès de méthyllithium est détruit par addition d'éthanol, suivie d'une hydrolyse en milieu acide par addition d'une solution aqueuse d'acide chlorydrique à 10%. La phase organique est extraite trois fois à l'éther diéthylique. Les phases éthérées sont rassemblées, lavées avec une solution d'acide chlorhydrique à 10%, à l'eau et enfin avec une solution aqueuse saturée en NaHCO₃. La phase organique est ensuite lavée jusqu'à neutralité, séché sur MgSO₄ puis concentrée sous vide. Le produit est purifié par reprécipitation dans l'acétone. Le composé 5 est obtenu sous la forme d'un solide blanc (19,8 g; point de fusion de 61,7-62,8°C; rendement de 87%). Son analyse par infra-rouge et RMN du proton et du carbone 13 est la suivante.
IR (ν (cm⁻¹)): 3330; 3079; 2919; 2851; 1642.
RMN ¹H (CDCl₃; δ (ppm)): 6,0-5,7 (m; 1 H); 5,1-4,7 (m; 2 H); 3,6 (t; 2 H); 2,2-1,9 (m; 2 H) et 1,7-1,2 (m; 37 H dont 1 H échangeable à D₂O).
RMN ¹³C (CDCl₃; δ (ppm)): 139,3; 113,8; 62,8; 33,6-25,8 (19 CH₂).

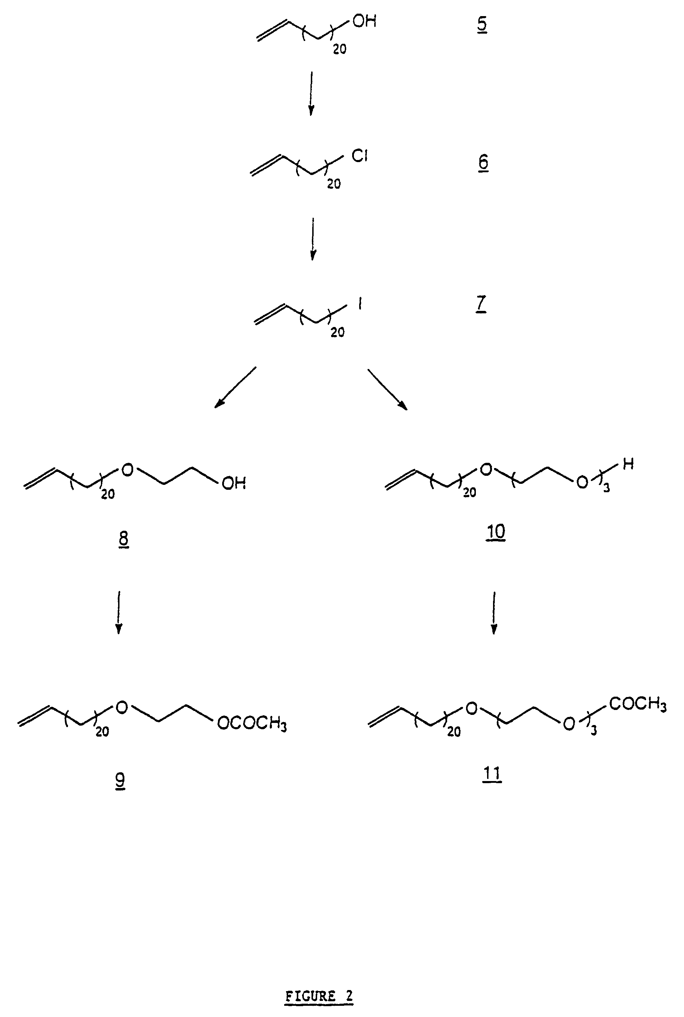

### 2) Introduction d'un éthylène glycol (figure 2).

### • Synthèse du dérivé chloré insaturé 6

L'alcool 5 obtenu précédemment (15 g; 46 mmol; 1 éq.) et de la pyridine (40,36 ml; 6 mmol; 0,1 éq.) sont introduits dans un ballon bicol de 250 ml, équipé d'une agitation magnétique et surmonté d'un réfrigérant ascendant. Du chlorure de thionyle (6 ml; 70 mmol; 1,5 éq.) est alors ajouté goutte à goutte. Le milieu réactionnel est agité pendant 1 h puis est porté au reflux jusqu'à la disparition complète de la bande OH (suivie par spectroscopie infra-rouge). Le milieu réactionnel est ensuite hydrolysé puis extrait trois fois à l'éther diéthylique. Les phases éthérées sont rassemblées, lavées avec une solution d'acide chlorhydrique à 10%, à l'eau, puis avec une solution saturée en NaHCO₃. La phase éthérée est ensuite lavée jusqu'à neutralité, séchée sur MgSO₄ et concentrée sous vide. Le composé 6 est obtenu sous la forme d'un solide jaune, puis est purifié par chromatographie sur silice (éluant: éther de pétrole/éther, v/v 70/30); un solide blanc est obtenu (14 g; point de fusion de 34,1-34,9°C; rendement de 75%). Son analyse par infra-rouge et RMN du proton et du carbone 13 est la suivante.
IR (dispersion dans KBr) ν (cm⁻¹): 3076; 2917; 2849; 1641.
RMN ¹H (CDCl₃; δ (ppm)): 6,0-5,7 (m; 1 H); 5,1-4,7 (m; 2 H); 3,5-3,3 (t; 2 H); 2,2-1,9 (m; 2 H) et 1,7-1,2 (m; 36 H).
RMN ¹³C (CDCl₃, δ (ppm)): 139,2; 113,8; 33,8-25,6 (20 CH₂).

### • Synthèse du dérivé iodé insaturé 7

Dans un ballon de 250 ml, le composé chloré insaturé 6 (10,6g; 32 mmol) et de l'iodure de sodium (22 g; 140 mmol; 4 éq.) sont solubilisés dans de l'acétone (40 ml). La solution est alors portée au reflux pendant 18 h. Le milieu réactionnel est ensuite extrait à l'éther diéthylique, les phases éthérées sont rassemblées puis lavées à l'eau, séchées sur MgSO₄ et concentrées sous vide. Le produit est purifié par des précipitations dans l'acétone. Le composé 7 est obtenu sous la forme d'un solide jaune (11g; point de fusion de 41,1-42,0°C; rendement de 81%). Son analyse par infra-rouge et RMN du proton et du carbone 13 est la suivante.
IR (dispersion dans KBr) ν (cm⁻¹): 3076; 2917; 2849; 1641.
RMN ¹H (CDCl₃; δ ppm)): 6,0-5,7 (m; 1 H); 5,1-4,7 (m; 2 H); 3,2-3,0 (t; 2 H); 2,2-1,9 (m; 2 H) et 1,7-1,2 (m; 36 H).
RMN ¹³C (CDCl₃; δ (ppm)): 139,2; 113,8; 33,8-25,6 (20 CH₂).

### • Synthèse de l'alcool insaturé 8

Une solution d'éthylène glycol (11,5 g; 180 mmol; 10 éq.) et de soude préalablement réduite en poudre (3,7 g; 90 mmol; 5 éq.) dans 20 ml de THF anhydre est portée au reflux durant 30 minutes. Le composé iodé 7 (8 g; 18 mmol; 1 éq) et du tétrabutyl-hydrogénosulfate d'ammonium (0,62 g; 1,8 mmol; 0,1 éq.) sont ajoutés. Le milieu réactionnel est ensuite porté au reflux durant 72 h. Après retour à température ambiante, une solution aqueuse d'acide chlorhydrique (10%, 50 ml) est introduite. Le milieu réactionnel est ensuite extrait trois fois à l'éther diéthylique; les phases éthérées sont rassemblées, lavées deux fois avec une solution d'acide chlorhydrique à 10%, à l'eau, puis avec une solution saturée en NaHCO₃. La phase éthérée est ensuite lavée jusqu'à neutralité, séchée sur MgSO₄ et concentrée sous vide. Le solide obtenu est reprécipité dans le dichlorométhane, puis purifié par chromatographie sur silice (éluant: dichlorométane/acétate d'éthyle; v/v: 30/70). Le composé 8 est obtenu sous la forme d'un solide blanc (1,4 g, point de fusion de 61,2-62,4°C; rendement de 21%). Son analyse par infra-rouge et RMN du proton et du carbone 13 est la suivante.
IR (dispersion dans KBr) ν (cm⁻¹): 3330; 3080; 2917; 2849; 1643.
RMN ¹H (CDCl₃; δ (ppm)): 6,0-5,7 (m; 1 H); 5,1-4,7 (m; 2 H); 3,75-3,65 (m; 2H) 3,55-3,4 (m; 4 H); 2,2-1,9 (m; 2 H) et 1,7-1,0 (m; 37 H dont 1 H échangeable à D₂O).
RMN ¹³C (CDCl₃; δ (ppm)): 139,2; 113,8; 71,7 (2 CH₂); 63,7; 33,6-25,8 (19 CH₂).

### • Synthèse de l'alcool insaturé protégé sous forme d'ester 9

Dans un ballon bicol de 100 ml, l'alcool insaturé 8 (0,9 g; 2,7 mmol) est mis en suspension dans du dichlorométhane (10 ml) et de la triéthylamine (0,6 ml; 5,4 mmol; 2 éq.). Le milieu réactionnel est refroidi à 0°C puis du chlorure d'acétyle (0,5 ml; 4 mmol; 1,5 éq.) est ajouté goutte à goutte à l'aide d'une seringue. Le milieu réactionnel est agité pendant 15 minutes à 0°C, puis 1 h 30 à température ambiante. Il est ensuite hydrolysé, puis extrait trois fois à l'éther diéthylique. Les phases éthérées sont rassemblées, lavées avec une solution d'acide chlorhydrique à 10%, à l'eau, puis avec une solution saturée en NaHCO₃. La phase éthérée est alors lavée jusqu'à neutralité, séchée sur MgSO₄ et concentrée sous vide. Le composé 9 est obtenu sous la forme d'un solide blanc (0,9 g; rendement de 100%). Son analyse par infra-rouge et RMN du proton et du carbone 13 est la suivante.
IR (dispersion dans KBr) ν (cm⁻¹): 3080; 2917; 2849; 1742; 1643.
RMN ¹H (CDCl₃; δ (ppm)): 6,0-5,7 (m; 1 H); 5,1-4,7 (m; 2 H); 4,25-4,15 (m; 2H); 3,60-3,50 (t; 2 H); 3,45-3,35 (t; 2 H); 2,2-1,9 (m; 5 H) et 1,7-1,0 (m; 36 H).
RMN ¹³C (CDCl₃; δ (ppm)): 172,0; 139,2; 113,8; 71,5; 68,5; 63,7; 33,6-25,8 (19 CH₂); 21,0.

### 3) Introduction de trois motifs éthylène glycol (figure 2).

A partir du dérivé iodé insaturé 7 obtenu ci-dessus et de triéthylèneglycol, un alcool insaturé 10 est obtenu, puis estérifié pour obtenir le produit 11, et ce selon les mêmes protocoles qu'exposés ci-dessus.

L'analyse du produit 10 par infra-rouge et RMN du proton et du carbone 13 est la suivante. IR (dispersion dans KBr) ν (cm⁻¹): 3380; 3079; 2919; 2850; 1641. RMN ¹H (CDCl₃; δ (ppm)): 5,9-5,7 (m; 1 H); 5,1-4,7 (m; 2 H); 4,3-4,2 (t; 2H); 3,7-3,4 (m; 10 H); 3,4-3,3 (t; 2 H); 2,2-1,9 (m; 2 H) et 1,7-1,0 (m; 37 H dont 1 H échangeable à D₂O). RMN ¹³C (CDCl₃; δ (ppm)): 139,3; 114,0; 71,6-68,2 (6 CH₂); 63,6; 33,6-25,8 (19 CH₂).

L'analyse du produit 11 par infra-rouge et RMN du proton et du carbone 13 est la suivante. IR (dispersion dans KBr) ν (cm⁻¹): 3079; 2919; 2850; 1740; 1641. RMN ¹H (CDCl₃; δ (ppm)): 5,9-5,7 (m; 1 H); 5,1-4,7 (m; 2 H); 4,3-4,2 (t; 2H); 3,7-3,4 (m; 10 H); 3,4-3,3 (t; 2 H); 2,2-1,9 (m; 5 H) et 1,7-1,0 (m; 36 H). RMN ¹³C (CDCl₃; δ (ppm)): 171,5; 139,3; 114,0; 71,6-68,3 (6 CH₂); 63,6; 33,6-25,8 (19 CH₂); 21,0.

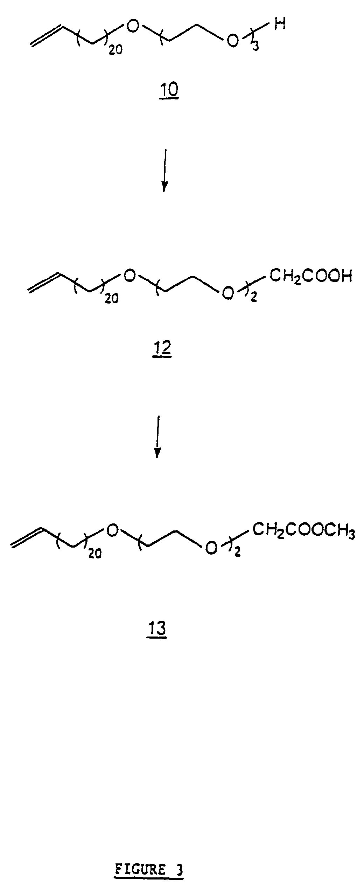

### 4) Préparation des esters insaturés (figure 3).

A partir de l'alcool insaturé 10 obtenu précédemment, l'acide et l'ester correspondants ont été préparés comme suit.

### • Préparation de l'acide 12

Dans un ballon tricol de 100 ml, l'alcool insaturé 10 (3 g; 6,6 mmol) est mis en suspension dans 10 ml d'acétone. A cette suspension est ajouté 5 ml de réactif de Jones 2M (BOWDEN *et al*., *J*. *Chem. Soc.,* 1946, *39*). La suspension est portée au reflux pendant 2 heures. Après retour à température ambiante, l'acétone est évaporée, le solide est filtré puis rincé 5 fois à l'eau et 3 fois à l'acétone refroidie à 0°C. Le solide est ensuite purifié par recristallisation dans un mélange THF/acétone (v/v: 9/1) pour donner le composé 12 sous la forme d'un solide blanc (2,9 g; rendement de 94%). Son analyse par infra-rouge et RMN du proton et du carbone 13 est la suivante.
IR (dispersion dans KBr) ν (cm⁻¹): 3370; 3080; 2917; 2849; 1707; 1643.
RMN ¹H (CDCl₃; δ (ppm)): 11,2 (s large; 1H);6,0-5,7 (m; 1 H); 5,1-4,7 (m; 2 H); 4,1-4,0 (s; 2 H) 3,6-3,3 (m; 10 H); 2,2-1,9 (m; 2 H) et 1,7-1,0 (m; 36 H).
RMN ¹³C (CDCl₃; δ (ppm)): 172,1; 139,1; 114,0; 71,6-68,7 (5 CH₂); 63,5; 33,6-25,8 (19 CH₂).

### • Préparation de l'ester 13

Dans un ballon tricol de 100 ml, l'acide 12 (2,9 g; 6,4 mmol) est solubilisé dans du toluène anhydre (7 ml) sous atmosphère inerte à 0°C. Du chlorure d'oxalyle (1,22 g; 9,6 mmol; 1,5 éq.) est introduit goutte à goutte puis le mélange est agité à température ambiante pendant deux heures. L'excès de réactif et le solvant sont ensuite évaporés sous vide. Le chlorure d'acyle est stocké temporairement sous argon. Du méthanol (6 ml; 128 mmol; 20 éq.), préalablement distillé sur chlorure de calcium, est ajouté lentement. Le milieu réactionnel est ensuite porté au reflux pendant 18 h avant d'être ramené à température ambiante, puis l'excès de méthanol est évaporé. Le milieu réactionnel est alors extrait trois fois à l'éther diéthylique. Les phases éthérées sont rassemblées, lavées avec une solution d'acide chlorhydrique à 10%, à l'eau et avec une solution saturée en NaHCO₃. La phase éthérée est ensuite lavée jusqu'à neutralité, séchée sur MgSO₄ et concentrée sous vide. Le composé 13 est obtenu sous la forme d'un solide blanc, puis est purifié par chromatographie sur silice (éluant : éther de pétrole/éther, 50/50 en volume). On obtient 300 mg d'un solide blanc (rendement de 10%). Son analyse par infra-rouge et RMN du proton et du carbone 13 est la suivante.
IR (dispersion dans KBr) ν (cm⁻¹): 3080; 2917; 2849; 1742; 1643.
RMN ¹H (CDCl₃; δ (ppm)): 6,0-5,7 (m; 1 H); 5,1-4,7 (m; 2 H); 4,1-4,0 (s; 2 H) 3,6-3,3 (m; 13 H); 2,2-1,9 (m; 2 H) et 1,7-1,0 (m; 36 H).
RMN ¹³C (CDCl₃; δ (ppm)): 171,8; 139,1; 114,0; 71,6-68,7 (5 CH₂); 63,6; 51,7; 33,6-25,8 (19 CH₂).

Il est bien entendu qu'en utilisant l'alcool 8 comprenant un seul motif éthylène glycol, les acides et les esters correspondants pourraient également être obtenus selon les mêmes protocoles que décrits ici à partir de l'alcool 10.

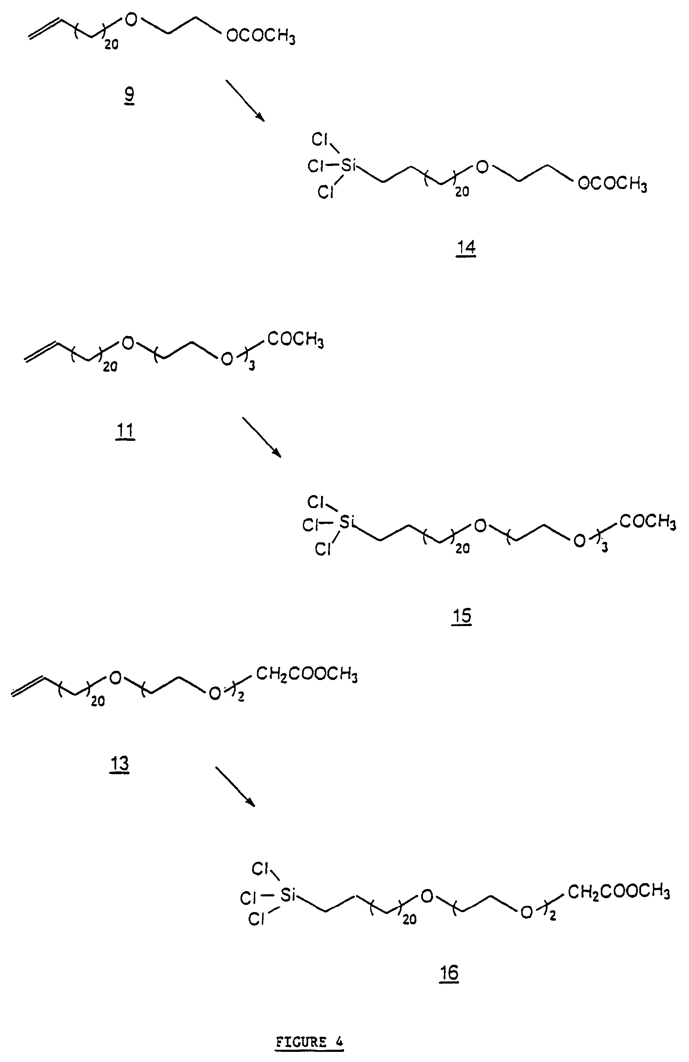

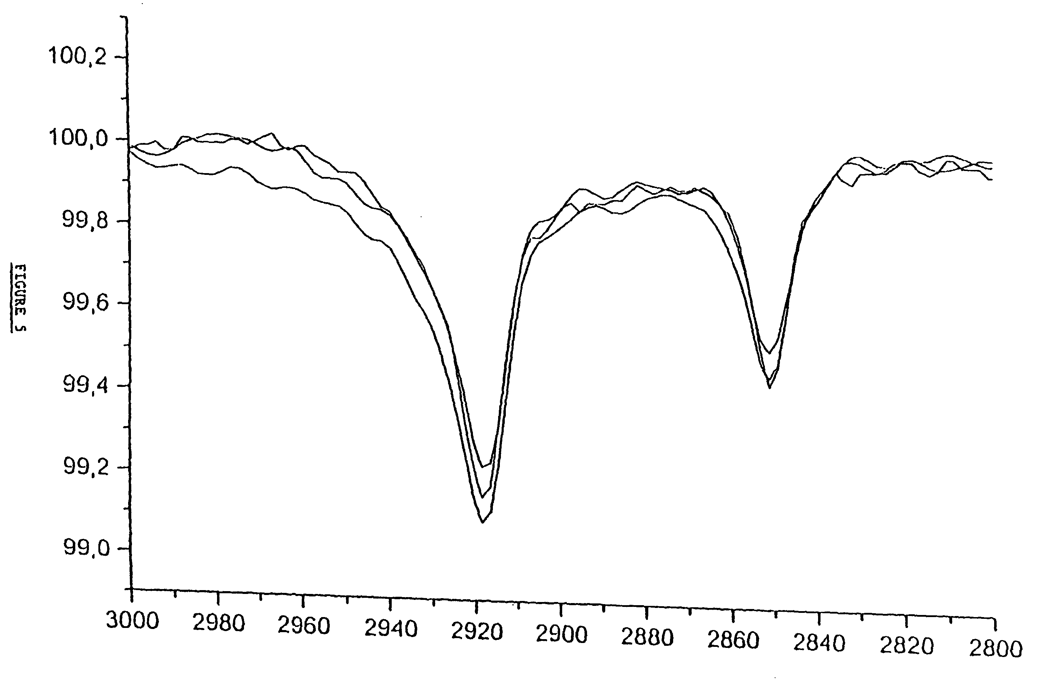

### 5) Silylation des précurseurs insaturés (figure 4).

Dans un schlenk sec, sous atmosphère inerte, l'ester 9 (150 mg; 0,33 mmol) est introduit. Du trichlorosilane fraîchement distillé (0,3 ml; 2,2 mmol; 6 éq.), du toluène anhydre (0,3 ml) ainsi qu'une goutte de catalyseur de Kärsted (PCO 72), commercialisé par ABCR (référence 68478-92-2), sont ajoutés. Le milieu réactionnel est alors porté à 40°C durant 2 h. Après retour à température ambiante, le toluène et l'excès de trichlorosilane sont évaporés sous pression réduite à l'aide d'une pompe à palette (pression de 0,5 mm de Hg). Le composé 14 est obtenu sous la forme d'un solide blanc et est stocké sous argon (rendement de 99%). Il s'agit d'un composé organosilicié de formule (I) selon la présente invention dans laquelle X₁, X₂ et X₃ représentent des atomes de chlore, n est égal à 22, i est égal à 0, k est égal à 1 et Z représente un groupement -COCH₃.

L'analyse du composé 14 par RMN du proton et du carbone 13 est la suivante.
RMN ¹H (CDCl₃; δ (ppm)): 4,25-4,15 (m; 2H); 3,60-3,50 (t; 2 H); 3,45-3,35 (t; 2 H); 2,2-1,9 (s; 3 H) et 1,7-1,0 (m; 42 H).
RMN ¹³C (CDCl₃; δ (ppm)): 171,0; 71,5; 68,5; 63,7; 31,9-22,3 (21 CH₂); 21.0.

En partant du composé 11 obtenu ci-avant et en utilisant le même protocole, on obtient le composé organosilicié 15 correspondant. Il s'agit d'un composé organosilicié de formule (I) selon la présente invention dans laquelle X₁, X₂ et X₃ représentent des atomes de chlore, n est égal à 22, i est égal à 0, k est égal à 3 et Z représente un groupement -COCH₃. Son analyse par RMN du proton et du carbone 13 est la suivante. RMN ¹H (CDCl₃; δ (ppm)): 4,3-4,2 (t; 2H); 3,7-3,4 (m; 10 H); 3,4-3,3 (t; 2 H); 2,2-1,9 (s; 3 H) et 1,7-0,9 (m; 42 H). RMN ¹³C (CDCl₃; δ (ppm)): 171,2; 71,6-68,1 (6 CH₂); 63,6; 31,9-22,3 (21 CH₂); 21,0.

En partant du composé 13 obtenu ci-avant et en utilisant le même protocole, on obtient le composé organosilicié 16 correspondant. Il s'agit d'un composé de formule (I) selon la présente invention dans laquelle X₁, X₂ et X₃ représentent des atomes de chlore, n est égal à 22, i est égal à 1, k est égal à 2 et Z représente un groupement -COOCH₃. Son analyse par RMN du proton et du carbone 13 est la suivante. RMN ¹H (CDCl₃; δ (ppm)): 4,1-4,0 (s; 2 H); 3,6-3,3 (m; 13 H); 1,7-0,9 (m; 42 H). RMN ¹³C (CDCl₃; δ (ppm)): 171,0; 71,6-63,8 (6 CH₂); 51,7; 31,9-22,3 (21 CH₂).

### EXEMPLE 2 : Silanisation d'un support solide à l'aide d'un composé organosilicié de formule (I) et obtention d'une monocouche autoassemblée organisée.

### 1) Silanisation du support solide.

Un disque de silicium, oxydé en surface, est utilisé comme substrat. Le disque est nettoyé selon la procédure suivante, afin d'éliminer les contaminants de sa surface et de l'hydrater :
- immersion dans un mélange sulfochromique fraîchement préparé (2,5 de K₂Cr₂O₄; 2,5 ml d'eau distillé; 50 ml d'acide sulfurique) pendant 10 minutes,
- sous une hotte à flux laminaire équipée de filtres à poussières, le disque est plongé dans de l'eau permutée et soumis aux ultrasons durant 20 minutes. Ce processus est répété deux fois avec des durées de sonication de 5 et 2 minutes respectivement,
- sous une hotte à flux laminaire équipée de filtres à poussières, le disque est introduit dans le réacteur de silanisation pour être séché, sous atmosphère inerte et filtrée. Le réacteur est plongé pendant 45 minutes dans un bain d'huile à 100°C puis est retiré du bain d'huile et sa température ramenée à 18°C.

Le composé organosilicié 14 obtenu dans l'exemple 1, fraîchement préparé en quantité voulue, sous atmosphère inerte, est solubilisé dans une fraction d'un mélange C₆H₁₂/CCl₄/CHCl₃ (v/v/v: 80/12/8). Le composé organosilicié 14 en solution est ensuite prélevé à la seringue puis introduit dans un schlenck contenant le reste du mélange de solvant dont le volume total a été calculé pour obtenir une solution de silanisation de dilution adéquate (entre 1.10⁻⁵ et 1.10⁻² mole/litre). Les solvants ont été préalablement séchés selon des procédures connues en elles-mêmes.

La solution de silanisation est introduite, avec une seringue, dans le réacteur et le disque de silicium reste immergé dans cette solution durant 16 h.

Le disque silanisé est retiré du réacteur, puis plongé dans du chloroforme (« HPLC grade ») et nettoyé aux ultrasons pendant 2 minutes. Ce processus est ensuite répété une deuxième fois.

### 2) Caractérisation de la surface modifiée.

On a obtenu ci-dessus un support solide dont la surface est modifiée par le composé 14. Le contrôle du greffage des composés organosiliciés est réalisé en utilisant la spectroscopie infra-rouge et la spectroscopie Raman confocale.

### 3) Libération des hydroxyles de surface.

Si nécessaire, les hydroxyles de surface peuvent être libérés en utilisant le protocole suivant : le disque silanisé est immergé dans une solution de KOH (0,5 M) dans un mélange eau/éthanol (v/v:1/1) durant 20 minutes. Le disque est ensuite nettoyé aux ultrasons durant 5 minutes dans de l'eau déminéralisée. Ce processus est renouvelé une fois dans l'eau puis une deuxième fois dans le chloroforme.

### 4) Caractérisation de la surface après libération des hydroxyles.

La figure 5 représente des spectres infra-rouge réalisés après trois expériences de greffage du composé 14 sur le support, selon le protocole donné en 1) et après saponification des esters de surface, comme indiqué ci-dessus. La transmittance figure en ordonnées et la fréquence (cm⁻¹) en abscisses. On remarque que les trois spectres se superposent, avec des pics caractéristiques d'un système organisé à 2917 et à 2850 cm⁻¹. Ainsi, on peut en conclure que le greffage du composé organosilicié sur le support est reproductible et donne lieu à la formation d'une monocouche autoassemblée organisée.

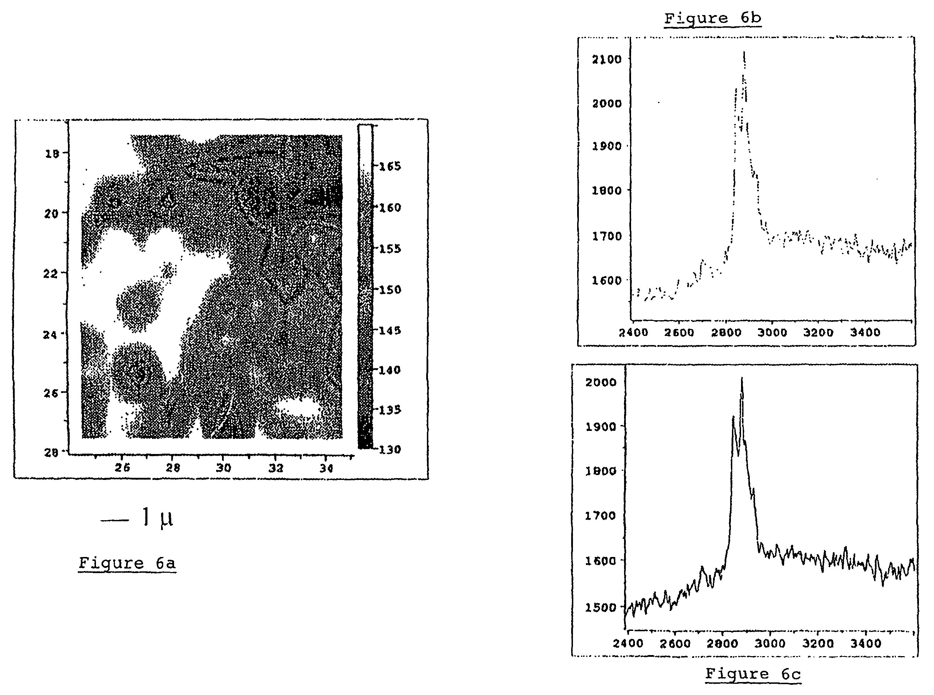

La figure 6 représente des études spectroscopiques Raman de la surface modifiée obtenue ci-dessus. La figure 6a est représentative de la densité de la surface du substrat greffé, les dimensions de la surface figurant en abscisses et en ordonnées (7 mm sur la figure correspondent à 1 µm sur le substrat) et l'échelle des densités étant graduée de 130 à 165 (unités arbitraires). Cette figure démontre l'homogénéité de la surface. Les figures 6b et 6c représentent les spectres Raman pris en deux points différents de la surface du substrat ; les ordonnées représentent des coups par seconde et la fréquence figure en abscisses.

Les spectres infra-rouge et Raman montrent donc sans ambiguïté l'homogénéité du film déposé sur le substrat ainsi que l'organisation des molécules sur la surface, caractéristique d'une monocouche autoassemblée organisée. Le spectre infra-rouge montre également que le greffage du film est parfaitement reproductible.

### EXEMPLE 3 : Autre exemple de silanisation d'un support solide à l'aide d'un composé organosilicié de formule (I).

Une lame de verre de microscope est utilisée comme substrat. La lame de verre est nettoyée par immersion dans une solution aqueuse d'Hellmanex® à 2% (commercialisée par Polylabo sous la référence 12240) pendant 2 h à 20°C, puis rinçage abondant à l'eau permutée.

Sous une hotte à flux laminaire équipée de filtres à poussières, la lame de verre est introduite dans le réacteur de greffage pour être séchée, sous atmosphère inerte et filtrée. Le réacteur est plongé pendant 45 minutes dans un bain d'huile à 100°C, puis est retiré du bain d'huile et sa température est ramenée à 18°C.

La solution de silanisation, préparée à l'aide du composé organosilicié 14 comme indiqué dans l'exemple 2, est introduite, avec une seringue, dans le réacteur et la lame de verre reste immergée dans cette solution durant 16 h. Le rinçage du substrat silanisé est effectué comme décrit dans l'exemple 2.

La caractérisation de la surface par spectroscopie infra-rouge et Raman démontrent là encore l'obtention d'une monocouche autoassemblée organisée sur le substrat.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Composés organosiliciés de formule (I) 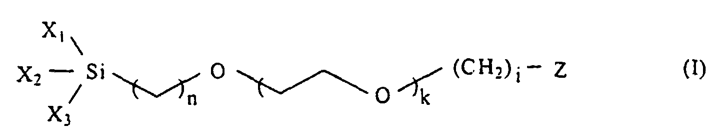 dans laquelle :
. n est compris entre 15 et 35,
. k est compris entre 0 et 100,
. i est un nombre entier supérieur ou égal à 0,
. X₁, X₂ et X₃, qui peuvent être identiques ou différents entre eux, sont sélectionnés dans le groupe constitué par les alkyles saturés en C₁ à C₆, linéaires ou ramifiés, et les groupements hydrolysables, au moins l'un de X₁, X₂ ou X₃ représentant un groupement hydrolysable, et
. si k = 0 et i = 0, alors Z représente un groupement R₁,
. si k = 0 et i ≥ 1, alors Z représente un groupement -OR₁, -OCOR₁, -NR₁R₂, -COOR₁, -CONR₁R₂, -SR₁ ou un atome d'halogène,
. si k ≥ 1 et i = 0, alors Z représente un groupement -R₁, -COR₁, -COOR₁, -CONR₁R₂, -CF₃ ou -(CF₂)ⱼCF₃, j étant compris entre 1 et 10,
. si k ≥ 1 et i ≥ 1, alors Z représente un groupement -OR₁, -OCOR₁, -NR₁R₂, -COOR₁, -CONR₁R₂, -SR₁, -CF₃, -(CF₂)ⱼCF₃, j étant tel que défini ci-dessus, ou un atome d'halogène,
. R₁ et R₂, qui peuvent être identiques ou différents, représentent un atome d'hydrogène, une chaîne hydrocarbonée éventuellement substituée, saturée ou insaturée et linéaire ou ramifiée, comprenant de 1 à 24 atomes de carbone, ou un groupement aromatique, à la condition que, lorsque k = i = 0 et n = 15, R₁ est différent du groupe -CH₂CF₃ et, lorsque k = i = 0 et n = 19, R₁ est différent du groupe -(CH₂)₆-C≡C-C≡CH.

2. Composés selon la revendication 1, **caractérisés en ce que** ledit groupement hydrolysable est sélectionné dans le groupe constitué par les atomes d'halogène, le groupement -N(CH₃)₂ et les groupements -OR, R étant un groupe alkyle saturé en C₁ à C₆, linéaire ou ramifié.

3. Composés selon la revendication 1 ou la revendication 2, **caractérisés en ce que** n est compris entre 20 et 25.

4. Composés selon l'une quelconque des revendications précédentes, **caractérisés en ce que** k est compris entre 0 et 5.

5. Composés selon l'une quelconque des revendications précédentes, **caractérisés en ce que** i est égal à 0 ou à 1.

6. Composés selon l'une quelconque des revendications précédentes, **caractérisé en ce que** X₁, X₂ et X₃ représentent des atomes de chlore, n est égal à 22, i est égal à 0, k est égal à 1 ou à 3 et Z représente un groupement -COCH₃.

7. Composés selon l'une quelconque des revendications 1 à 5, **caractérisés en ce que** X₁, X₂ et X₃ représentent des atomes de chlore, n est égal à 22, i est égal à 1, k est égal à 2 et Z représente un groupement -COOCH₃.

8. Procédé de préparation des composés de formule (I) selon l'une quelconque des revendications 1 à 6 dans laquelle i est différent de 1, qui comprend les étapes suivantes :
a) préparation d'un précurseur insaturé de formule (III) : 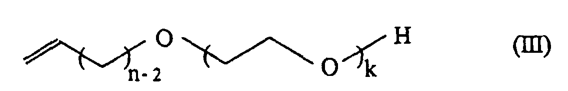 par réaction d'un diol de formule HO-(CH₂-CH₂-O)ₖ-H avec un composé insaturé de formule (II) : 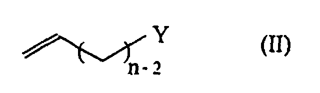 dans lesquelles Y représente un groupe nucléofuge et n et k sont tels que définis dans l'une quelconque des revendications 1 à 6 ;
b) obtention, par fonctionnalisation de l'extrémité hydroxyle du composé de formule (III), d'un précurseur insaturé de formule (IV) : 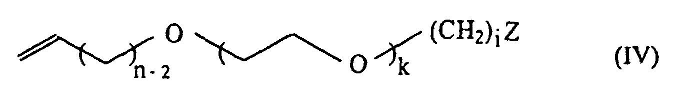 dans laquelle Z et i sont tels que définis dans l'une quelconque des revendications 1 à 6 ;
c) obtention, par hydrosilylation du précurseur insaturé de formule (IV) à l'aide d'un hydrogénosilane de formule HSiX₁X₂X₃, d'un composé silicié de formule (I) : 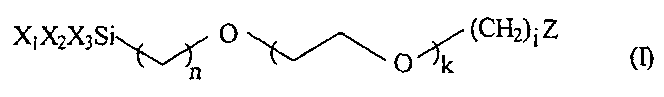 dans laquelle au moins l'un de X₁, X₂ et X₃ représente un atome d'halogène ; et
d) éventuellement, obtention d'un autre composé de formule (I) par substitution d'un ou de plusieurs des groupements X₁, X₂ et X₃ du composé obtenu à l'étape c) à l'aide de groupements X₁, X₂ et/ou X₃ tels que définis dans l'une quelconque des revendications 1 à 6.

9. Procédé de préparation des composés de formule (I) selon l'une quelconque des revendications 1 à 5 et 7 dans laquelle i est égal à 1, qui comprend les étapes suivantes :
a) préparation d'un précurseur insaturé de formule (III') : 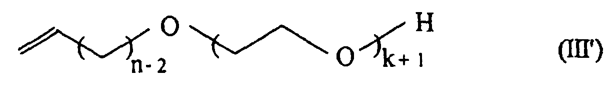 par réaction d'un composé insaturé de formule (II) telle que définie dans la revendication 6 avec un diol de formule HO-(CH₂-CH₂-O)ₖ₊₁-H, n et k étant tels que définis dans l'une quelconque des revendications 1 à 5 et 7 ;
b) obtention, par oxydation de l'extrémité hydroxyle du composé (III'), d'un précurseur insaturé de formule (IV') : 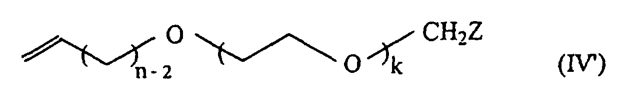 dans laquelle Z représente une fonction acide carboxylique ;
c) éventuellement, fonctionnalisation de l'extrémité acide carboxylique du composé de formule (IV') à l'aide d'un autre groupement Z tel que défini dans l'une quelconque des revendications 1 à 5 et 7 ;
d) obtention, par hydrosilylation du précurseur insaturé de formule (IV') à l'aide d'un hydrogénosilane de formule HSiX₁X₂X₃, d'un composé silicié de formule (I) : 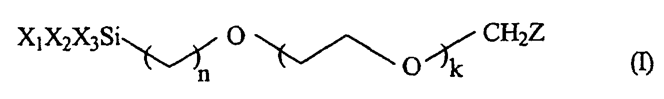 dans laquelle au moins l'un de X₁, X₂ et X₃ représente un atome d'halogène ; et
e) éventuellement, obtention d'un autre composé de formule (I) par substitution d'un ou de plusieurs des groupements X₁, X₂ et X₃ du composé obtenu à l'étape d) à l'aide de groupements X₁, X₂ et/ou X₃ tels que définis dans l'une quelconque des revendications 1 à 5 et 7.

10. Utilisation d'un composé de formule (I') : 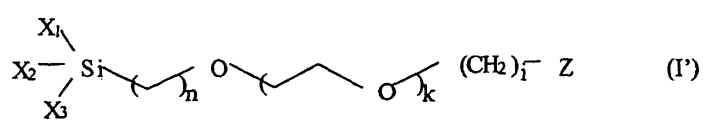 dans laquelle :
. n est compris entre 15 et 35,
. k est compris entre 0 et 100,
. i est un nombre entier supérieur ou égal à 0,
. X₁, X₂ et X₃, qui peuvent être identiques ou différents entre eux, sont sélectionnés dans le groupe constitué par les alkyles saturés en C₁ à C₆, linéaires ou ramifiés, et les groupements hydrolysables, au moins l'un de X₁, X₂ ou X₃ représentant un groupement hydrolysable, et
. si k = 0 et i = 0, alors Z représente un groupement R₁,
. si k = 0 et i ≥ 1, alors Z représente un groupement -OR₁, -OCOR₁, -NR₁R₂, -COOR₁, -CONR₁R₂, -SR₁ ou un atome d'halogène,
. si k ≥ 1 et i = 0, alors Z représente un groupement -R₁, -COR₁, -COOR₁, -CONR₁R₂, -CF₃ ou -(CF₂)ⱼCF₃, j étant compris entre 1 et 10,
. si k ≥ 1 et i ≥ 1, alors Z représente un groupement -OR₁, -OCOR₁, -NR₁R₂, -COOR₁, -CONR₁R₂, -SR₁, -CF₃, -(CF₂)ⱼCF₃, j étant tel que défini ci-dessus, ou un atome d'halogène,
. R₁ et R₂, qui peuvent être identiques ou différents, représentent un atome d'hydrogène, une chaîne hydrocarbonée éventuellement substituée, saturée ou insaturée et linéaire ou ramifiée, comprenant de 1 à 24 atomes de carbone, ou un groupement aromatique ; étant entendu que lorsque X₁ = X₂ = X₃ = Cl, que k = i = 0 et que n = 19, alors R₁ est différent du groupe -(CH₂)₆-C≡C-C≡CH,
pour former, à la surface d'un support solide, une monocouche autoassemblée organisée.

11. Support solide dont la surface est modifiée par une monocouche autoassemblée organisée, **caractérisé en ce que** ladite monocouche comprend un réseau d'au moins un composé organosilicié de formule (I') telle que définie dans la revendication 10.

12. Support selon la revendication 11, **caractérisé en ce que** ledit support solide est tel que sa surface présente, avant d'être modifiée, des groupements hydroxyles.

13. Support selon la revendication 12, **caractérisé en ce que** ledit support solide est sélectionné dans le groupe constitué par les verres, les céramiques, les métaux et les métalloïdes.

14. Procédé d'obtention d'un support solide selon l'une quelconque des revendications 11 à 13, **caractérisé en ce qu'**il comprend les étapes suivantes :
a) élimination des contaminants du support solide et hydratation et/ou hydroxylation de sa surface,
b) introduction, sous atmosphère inerte, d'au moins un composé organosilicié de formule (1') telle que définie dans la revendication 10 dans un mélange d'au moins deux solvants comprenant au moins un solvant hydrocarboné non polaire,
c) silanisation du support obtenu à l'étape a) par immersion dans la solution préparée à l'étape b), et
d) rinçage du support modifié obtenu à l'étape c) à l'aide d'un solvant.

15. Procédé selon la revendication 14, **caractérisé en ce que** l'étape a) eat effectuée, selon la nature du support solide, à l'aide d'un ou de plusieurs solvants et/ou oxydants et/ou hydroxylants, d'un détergent ou d'un traitement phutochimique à l'ozone.

16. Procédé selon la revendication 14 ou la revendication 15, **caractérisé en ce que** l'étape b) est réalisée dans un mélange d'au moins un solvant hydrocarboné non polaire et d'au moins un solvant polaire.

17. Procédé selon la revendication 16, **caractérisé en ce que** les proportions volumiques de solvant non polaire et de solvant polaire sont comprises entre 70/30 et 95/5.

18. Procédé selon l'une quelconque des revendications 14 à 17, **caractérisé en ce que** la concentration du composé organosilicié dans le mélange de solvants, à l'étape b) du procédé, est comprise entre 1.10⁻⁵ et 1.10⁻² mole/litre.

19. Utilisation d'un support solide selon l'une quelconque des revendications 11 à 13 pour la synthèse ou l'immobilisation de biomolécules par liaison covalente.

20. Procédé de synthèse de biomolécules sur un support solide selon l'une quelconque des revendications 11 à 13, **caractérisé en ce que** lesdites biomolécules sont constituées d'un enchaînement d'unités répétitives et **en ce que** ledit procédé comprend des étapes successives de greffage desdites unités répétitives, la première unité répétitive greffée portant une fonction réactive vis-à-vis des groupements 7, des composés organosiliciés présents sur le support solide.

21. Procédé d'immobilisation de biomolécules sur un support solide selon l'une quelconque des revendications 11 à 13, **caractérisé en ce qu'**il comprend une étape de greffage desdites biomolécules, qui portent des fonctions réactives vis-à-vis des groupements Z des composés organosiliciés, sur ledit support solide.

22. Procédé selon la revendication 20 ou la revendication 21, **caractérisé en ce qu'**il est précédé, dans le cas où la fonction terminale Z des composés organosiliciés est un groupement -OCOR₁ dans lequel R₁ est tel que défini dans la revendication 10, ou un groupement -COOR₁ dans lequel R₁ est tel que défini dans la revendication 10 et est différent d'un atome d'hydrogène, d'une étape de déprotection de la fonction alcool ou acide carboxylique correspondanté par un traitement chimique approprié.

## Patentansprüche

1. Siliciumorganische Verbindungen der Formel (I) 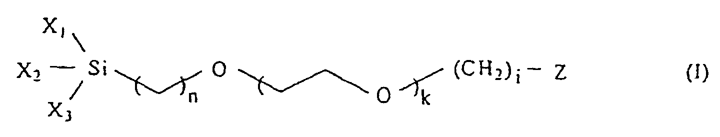 worin
- n zwischen 15 und 35 liegt;
- k zwischen 0 und 100 liegt;
- i eine ganze Zahl größer oder gleich 0 ist;
- X₁, X₂ und X₃, die gleich oder voneinander verschieden sein können, aus der Gruppe bestehend aus gesättigten, linearen oder verzweigten C₁-C₆-Alkylgruppen und hydrolysierbaren Gruppen ausgewählt sind, wobei mindestens eine der Gruppen X₁, X₂ und X₃ eine hydrolysierbare Gruppe darstellt; und
- wenn k=0 und i=0, Z dann eine Gruppe R₁ darstellt;
- wenn k=0 und i ≥ 1, Z dann eine Gruppe -OR₁, -OCOR₁, -NR₁R₂, -COOR₁, -CONR₁R₂, -SR₁ oder ein Halogenatom darstellt;
- wenn k ≥ 1 und i=0, Z dann eine Gruppe -R₁, COR₁, COOR₁, -CONR₁R₂, -CF₃ oder -(CF₂)ⱼCF₃ darstellt, wobei j zwischen 1 und 10 liegt;
- wenn k ≥ 1 und i ≥ 1, Z dann eine Gruppe -OR₁, -OCOR₁, -NR₁R₂, -COOR₁, -CONR₁R₂, -SR₁, -CF₃, -(CF₂)ⱼCF₃, wobei j wie oben definiert ist, oder ein Halogenatom darstellt;
- R₁ und R₂, die gleich oder voneinander verschieden sein können, ein Wasserstoffatom, eine gegebenenfalls substituierte, gesättigte oder ungesättigte und lineare oder verzweigte Kohlenwasserstoffkette, die 1 bis 24 Kohlenstoffatome umfasst, oder eine aromatische Gruppe darstellen, mit der Maßgabe, dass, wenn k=i=0 und n=15, R₁ dann von der Gruppe -CH₂CF₃ verschieden ist, und, wenn k=i=0 und n=19, R₁ sich dann von der Gruppe -(CH₂)₆-C≡C-C≡CH unterscheidet.

2. Verbindungen nach Anspruch 1, **dadurch gekennzeichnet, dass** die hydrolysierbare Gruppe aus der Gruppe bestehend aus Halogenatomen, der Gruppe -N(CH₃)₂ und den Gruppen -OR, worin R eine gesättigte, lineare oder verzweigte C₁-C₆-Alkylgruppe ist, ausgewählt ist.

3. Verbindungen nach Anspruch 1 oder Anspruch 2, **dadurch gekennzeichnet, dass** n zwischen 20 und 25 liegt.

4. Verbindungen nach einem der vorangehenden Ansprüche, **dadurch gekennzeichnet, dass** k zwischen 0 und 5 liegt.

5. Verbindungen nach einem der vorangehenden Ansprüche, **dadurch gekennzeichnet, dass** i gleich 0 oder gleich 1 ist.

6. Verbindungen nach einem der vorangehenden Ansprüche, **dadurch gekennzeichnet, dass** X₁, X₂ und X₃ Chloratome darstellen, n gleich 22 ist, i gleich 0 ist, k gleich 1 oder 3 ist und Z eine Gruppe -COCH₃ darstellt.

7. Verbindungen nach einem der Ansprüche 1 bis 5, **dadurch gekennzeichnet, dass** X₁, X₂ und X₃ Chloratome darstellen, n gleich 22 ist, i gleich 1 ist, k gleich 2 ist und Z die Gruppe -COOCH₃ darstellt.

8. Verfahren zur Herstellung der Verbindungen der Formel (I) nach einem der Ansprüche 1 bis 6, worin i von 1 verschieden ist, umfassend die folgenden Stufen:
a) Herstellung eines ungesättigten Vorläufers der Formel (III): 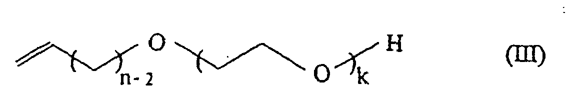 durch Umsetzung eines Diols der Formel HO-(CH₂-CH₂-O)ₖ-H mit einer ungesättigten Verbindung der Formel (II): 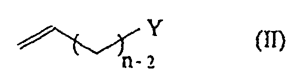 worin Y eine Abgangsgruppe darstellt und n und k wie in einem der Ansprüche 1 bis 6 definiert sind;
b) Erhalt eines ungesättigten Vorläufers der Formel (IV) durch Funktionalisierung des Hydroxyl-Endes der Verbindung der Formel (III) : 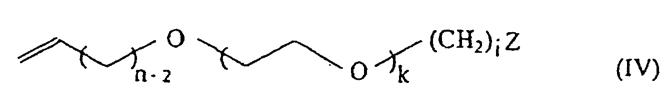 worin Z und i wie in einem der Ansprüche 1 bis 6 definiert sind;
c) durch Hydrosilylierung des ungesättigten Vorläufers der Formel (IV) mit Hilfe eines Hydrogensilans der Formel HSiX₁X₂X₃, Erhalt einer Siliciumverbindung der Formel (I) : 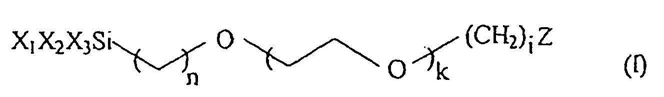 worin mindestens eines der X₁, X₂ und X₃ ein Halogenatom darstellt, und
d) gegebenenfalls Erhalt einer anderen Verbindung der Formel (I) durch Substitution einer oder mehrerer der Gruppen X₁, X₂ und X₃ der in Stufe c) erhaltenen Verbindung mit Hilfe von Gruppen X₁, X₂ und/oder X₃, wie sie in einem der Ansprüche 1 bis 6 definiert sind.

9. Verfahren zur Herstellung der Verbindungen der Formel (I) nach einem der Ansprüche 1 bis 5 und 7, worin i gleich 1 ist, umfassend die folgenden Stufen:
a) Herstellung eines ungesättigten Vorläufers der Formel (III'): 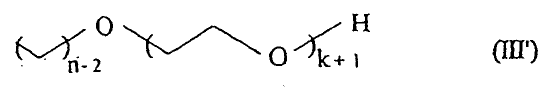 durch Umsetzung einer ungesättigten Verbindung der Formel (II), wie sie in Anspruch 6 definiert ist, mit einem Diol der Formel HO(CH₂-CH₂-O)ₖ₊₁-H, wobei n und k wie in einem der Ansprüche 1 bis 5 und 7 definiert sind;
b) durch Oxidation des Hydroxylendes der Verbindung (III') Erhalt eines ungesättigten Vorläufers der Formel (IV'): 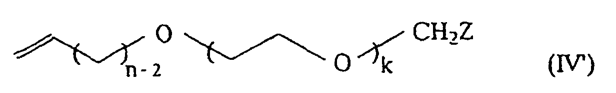 worin Z eine Carbonsäurefunktion darstellt;
c) gegebenenfalls Funktionalisierung des Carbonsäureendes der Verbindung der Formel (IV') mit Hilfe einer anderen Gruppe Z, wie sie in einem der Ansprüche 1 bis 5 und 7 definiert ist;
d) durch Hydrosilylierung des ungesättigten Vorläufers der Formel (IV') mit Hilfe eines Hydrogensilans der Formel HSiX₁X₂X₃ Erhalt einer Siliciumverbindung der Formel (I): 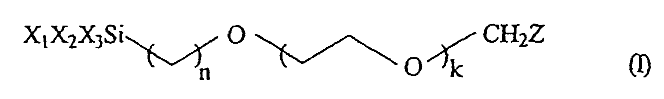 worin mindestens eine der X₁, X₂ und X₃ ein Halogenatom darstellt und
e) gegebenenfalls Erhalt einer anderen Verbindung der Formel (I) durch Substitution einer oder mehrerer der Gruppen X₁, X₂ und X₃ der in Stufe d) erhaltenen Verbindung mit Hilfe der Gruppen X₁, X₂ und/oder X₃, wie sie in einem der Ansprüche 1 bis 5 und 7 definiert sind.

10. Verwendung einer Verbindung der Formel (I'): 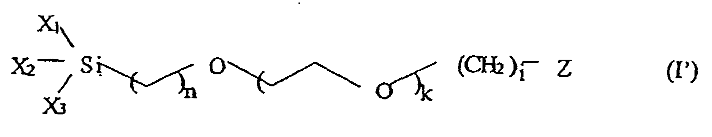 worin
- n zwischen 15 und 35 liegt;
- k zwischen 0 und 100 liegt;
- i eine ganze Zahl größer oder gleich 0 ist;
- X₁, X₂ und X₃, die gleich oder voneinander verschieden sein können, aus der Gruppe bestehend aus gesättigten, linearen oder verzweigten C₁-C₆-Alkylgruppen und hydrolysierbaren Gruppen ausgewählt sind, wobei mindestens eine der Gruppen X₁, X₂ und X₃ eine hydrolysierbare Gruppe darstellt; und
- wenn k=0 und i=0, Z dann eine Gruppe R₁ darstellt;
- wenn k=0 und i ≥ 1, Z dann eine Gruppe -OR₁, -OCOR₁, -NR₁R₂,
-COOR₁, -CONR₁R₂, -SR₁ oder ein Halogenatom darstellt;
- wenn k ≥ 1 und i=0, Z dann eine Gruppe -R₁, -COR₁, -COOR₁, -CONR₁R₂, -CF₃ oder -(CF₂)ⱼCF₃ darstellt, wobei j zwischen 1 und 10 liegt;
- wenn k ≥ 1 und i ≥ 1, Z dann eine Gruppe -OR₁, -OCOR₁, -NR₁R₂, -COOR₁, -CONR₁R₂, -SR₁, -CF₃, -(CF₂)ⱼCF₃, wobei j wie oben definiert ist, oder ein Halogenatom darstellt;
- R₁ und R₂, die gleich oder voneinander verschieden sein können, ein Wasserstoffatom, eine gegebenenfalls substituierte, gesättigte oder ungesättigte und lineare oder verzweigte Kohlenwasserstoffkette, die 1 bis 24 Kohlenstoffatome umfasst, oder eine aromatische Gruppe darstellen, mit der Maßgabe, dass, wenn X₁=X₂=X₃=Cl, k=i=0 und n=19, R₁ dann von der Gruppe -(CH₂)₆-C≡C-C≡CH verschieden ist;
zur Bildung einer geordneten selbst organisierten Monoschicht an der Oberfläche eines festen Trägers.

11. Fester Träger, dessen Oberfläche durch eine geordnete selbst organisierte Monoschicht modifiziert ist, **dadurch gekennzeichnet, dass** die Monoschicht ein Harz mindestens einer siliciumorganischen Verbindung der Formel (I'), wie sie in Anspruch 10 definiert ist, umfasst.

12. Träger nach Anspruch 11, **dadurch gekennzeichnet, dass** der feste Träger so ist, dass seine Oberfläche, bevor er modifiziert wurde, Hydroxylgruppen aufweist.

13. Träger nach Anspruch 12, **dadurch gekennzeichnet, dass** der feste Träger aus der Gruppe bestehend aus Gläsern, Keramik, Metallen und Metalloiden ausgewählt ist.

14. Verfahren zum Erhalt eines festen Trägers nach einem der Ansprüche 11 bis 13, **dadurch gekennzeichnet, dass** es die folgenden Stufen umfasst:
a) Eliminierung von Verunreinigungen des festen Trägers und Hydratisierung und/oder Hydroxylierung seiner Oberfläche;
b) Einführung mindestens einer siliciumorganischen Verbindung der Formel (I'), wie sie in Anspruch 10 definiert ist, in ein Gemisch aus mindestens zwei Lösungsmitteln, die mindestens ein nicht polares Kohlenwasserstofflösungsmittel umfassen, unter inerter Atmosphäre;
c) Silanisierung des in Stufe a) erhaltenen Trägers durch Eintauchen in die in Stufe b) hergestellte Lösung und
d) Spülen des modifizierten Trägers, der in Stufe c) erhalten wurde, mit Hilfe eines Lösungsmittels.

15. Verfahren nach Anspruch 14, **dadurch gekennzeichnet, dass** die Stufe a) entsprechend der Natur des festen Trägers mit Hilfe eines oder mehrerer Lösungsmittel und/oder Oxidationsmittel und/oder Hydroxylierungsmittel, eines Detergenzes oder einer photochemischen Behandlung mit Ozon durchgeführt wird.

16. Verfahren nach Anspruch 14 oder Anspruch 15, **dadurch gekennzeichnet, dass** die Stufe b) in einem Gemisch aus mindestens einem nicht polaren Kohlenwasserstofflösungsmittel und mindestens einem polaren Lösungsmittel durchgeführt wird.

17. Verfahren nach Anspruch 16, **dadurch gekennzeichnet, dass** die Volumenverhältnisse des nicht polaren Lösungsmittels und des polaren Lösungsmittels zwischen 70/30 und 95/5 liegen.

18. Verfahren nach einem der Ansprüche 14 bis 17, **dadurch gekennzeichnet, dass** die Konzentration der siliciumorganischen Verbindung in dem Lösungsmittelgemisch in Stufe b) des Verfahrens zwischen 1 x 10⁻⁵ und 1 x 10⁻² mol/l liegt.

19. Verwendung eines festen Trägers nach einem der Ansprüche 11 bis 13 zur Synthese oder Immobilisierung von Biomolekülen durch kovalente Bindung.

20. Verfahren zur Synthese von Biomolekülen auf einem festen Träger nach einem der Ansprüche 11 bis 13, **dadurch gekennzeichnet, dass** die Biomoleküle aus einer Verknüpfung von Repetiereinheiten bestehen und dass das Verfahren sukzessive Stufen der Pfropfung der genannten Repetiereinheiten umfasst, wobei die erste gepfropfte Repetiereinheit eine Funktion umfasst, welche gegenüber den Gruppen Z der siliciumorganischen Verbindungen, die auf dem festen Träger vorliegen, reaktiv sind.

21. Verfahren zur Immobilisierung von Biomolekülen auf einem festen-Träger nach einem der Ansprüche 11 bis 13, **dadurch gekennzeichnet, dass** es eine Stufe der Pfropfung der Biomoleküle, die Funktionen tragen, die gegenüber den Gruppen Z der siliciumorganischen Verbindungen reaktiv sind, an den festen Träger umfasst.

22. Verfahren nach Anspruch 20 oder Anspruch 21, **dadurch gekennzeichnet, dass** es in dem Fall, in dem die terminale Funktion Z der siliciumorganischen Verbindungen eine Gruppe -OCOR₁, in der R₁ wie in Anspruch 10 definiert ist, oder eine Gruppe -COOR₁, in der R₁ wie in Anspruch 10 definiert ist und sich von einem Wasserstoffatom unterscheidet, ist, eine vorausgeschaltete Stufe der Entschützung der Alkoholfunktion oder der Carbonsäurefunktion umfasst, die durch eine entsprechende chemische Behandlung erfolgt.

## Claims

1. Organosilicon compounds of formula (I) 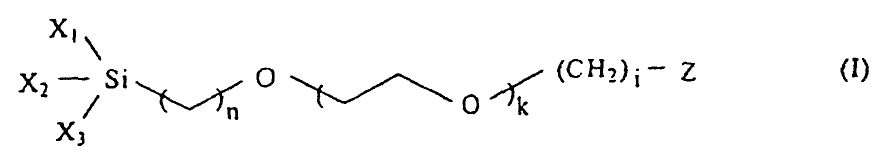 in which:
. n is between 15 and 35,
. k is between 0 and 100,
. i is an integer number greater than or equal to 0,
. X₁, X₂, and X₃, which may be identical or different from each other, are selected from the group consisting of linear or branched C₁ to C₆ saturated alkyls and hydrolysable groups, at least one of X₁, X₂ or X₃ representing a hydrolysable group, and
. if k = 0 and i = 0, then Z represents an R₁ group,
. if k = 0 and i ≥ 1, then Z represents an -OR₁, -OCOR₁, -NR₁R₂, -COOR₁, -CONR₁R₂ or -SR₁ group or a halogen atom,
. if k ≥ 1 and i = 0, then Z represents an -R₁, -COR₁, -COOR₁, - CONR₁R₂, -CF₃ or -(CF₂)ⱼCF₃ group, j being between 1 and 10,
. if k ≥ 1 and i ≥ 1, then Z represents an -OR₁, -OCOR₁, -NR₁R₂, -COOR₁, -CONR₁R₂, -SR₁, -CF₃ or -(CF₂)ⱼCF₃ group, j being as defined above, or a halogen atom,
. R₁ and R₂, which may be identical or different, represent a hydrogen atom, a hydrocarbon chain, possibly substituted, saturated or unsaturated and linear or branched, comprising 1 to 24 carbon atoms, or an aromatic group, provided that, when k = i = 0 and n = 15, R₁ is different from the group -CH₂CF₃ and, when k = i = 0 and n = 19, R₁ is different from the group -(CH₂)₆-C≡C-C≡CH.

2. Compounds according to Claim 1, **characterised in that** the said hydrolysable group is selected from the group consisting of halogen atoms, the group -N(CH₃)₂ and the -OR groups, R being a linear or branched C₁ to C₆ saturated alkyl group.

3. Compounds according to Claim 1 or Claim 2, **characterised in that** n is between 20 and 25.

4. Compounds according to any one of the preceding claims, **characterised in that** k is between 0 and 5.

5. Compounds according to any one of the preceding claims, **characterised in that** i is equal to 0 or 1.

6. Compounds according to any one of the preceding claims, **characterised in that** X₁, X₂ and X₃ represent chlorine atoms, n is equal to 22, i is equal to 0, k is equal to 1 or 3 and Z represents a -COCH₃ group.

7. Compounds according to any one of Claims 1 to 5, **characterised in that** X₁, X₂ and X₃ represent chlorine atoms, n is equal 22, i is equal to 1, k is equal to 2 and Z represents a -COOCH₃ group.

8. Method of preparing compounds of formula (I) according to any one of Claims 1 to 6, in which i is different from 1, which comprises the following steps:
a) preparation of an unsaturated precursor of formula (III): 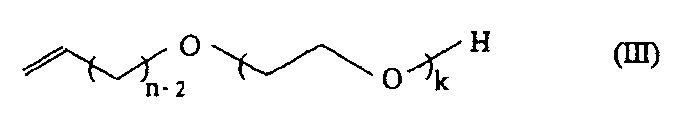 by reaction of a diol of formula HO-(CH₂-CH₂-O)ₖ-H with an unsaturated compound of formula (II): 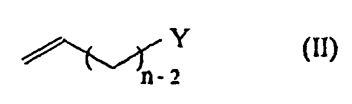 in which Y represents a nucleofugal group and n and k are as defined in any one of Claims 1 to 6;
b) obtaining, by functionalisation of the hydroxyl end of the compound of formula (III), an unsaturated precursor of formula (IV): 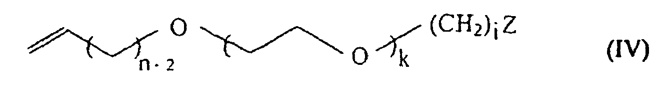 in which Z and i are as defined in any one of Claims 1 to 6;
c) obtaining, by hydrosilylation of the unsaturated precursor of formula (IV) by means of a hydrogenosilane of formula HSiX₁X₂X₃, a silicon compound of formula (I): 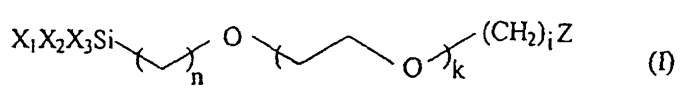 in which at least one of X₁, X₂ and X₃ represents a halogen atom; and
d) possibly obtaining another compound of formula (I) by substituting one or more of the groups X₁, X₂ and X₃ of the compound obtained at step c) by means of the groups X₁, X₂ and/or X₃ as defined in any one of Claims 1 to 6.

9. Method of preparing compounds of formula (I) according to any one of Claims 1 to 5 and 7 in which i is equal to 1, which comprises the following steps:
a) preparing an unsaturated precursor of formula (III'): 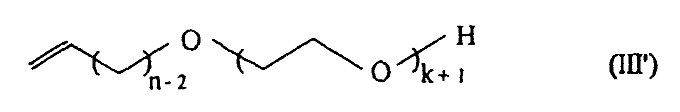 by reaction of an unsaturated compound of formula (II) as defined in Claim 6 with a diol of formula HO-(CH₂-CH₂-O)ₖ₊₁-H, n and k being as defined in any one of Claims 1 to 5 and 7;
b) obtaining, by oxidation of the hydroxyl end of the compound (III'), an unsaturated precursor of formula (IV'): 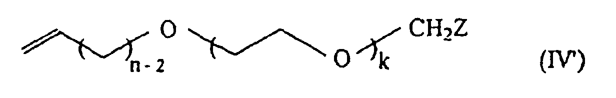 in which Z represents a carboxyl acid function;
c) possibly functionalisation of the carboxyl acid end of the compound of formula (IV') by means of another Z group as defined in any one of Claims 1 to 5 and 7;
d) obtaining, by hydrosilylation of the unsaturated precursor of formula (IV') by means of a hydrogenosilane of formula HSiX₁X₂X₃, a silicon compound of formula (I) : 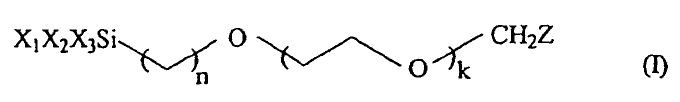 in which at least one of X₁, X₂ and X₃ represents a halogen atom; and
e) possibly obtaining another compound of formula (I) by substituting one or more of the groups X₁, X₂ and X₃ of the compound obtained at step d) by means of groups X₁, X₂ and/or X₃ as defined in any one of Claims 1 to 5 and 7.

10. Use of a compound of formula (I'): 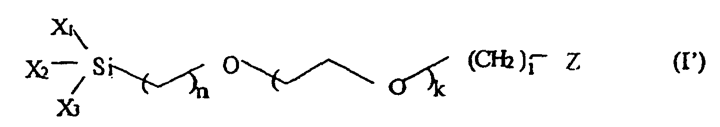 in which:
. n is between 15 and 35,
. k is between 0 and 100,
. i is an integer number greater than or equal to 0,
. X₁, X₂, and X₃, which may be identical or different from each other, are selected from the group consisting of linear or branched C₁ to C₆ saturated alkyls and the hydrolysable groups, at least one of X₁, X₂ or X₃ representing a hydrolysable group, and
. if k = 0 and i = 0, then Z represents an R₁ group,
. if k = 0 and i ≥ 1, then Z represents an -OR₁, -OCOR₁, -NR₁R₂, -COOR₁, -CONR₁R₂ or -SR₁ group or a halogen atom,
. if k ≥ 1 and i = 0, then Z represents an -R₁, -COR₁, -COOR₁, - CONR₁R₂, -CF₃ or -(CF₂)ⱼCF₃ group, j being between 1 and 10,
. if k ≥ 1 and i ≥ 1, then Z represents a -OR₁, -OCOR₁, -NR₁R₂, -COOR₁, -CONR₁R₂, -SR₁, -CF₃ or -(CF₂)ⱼCF₃ group, j being as defined above, or a halogen atom,
. R₁ and R₂, which may be identical or different, represent a hydrogen atom, a hydrocarbon chain, possibly substituted, saturated or unsaturated and linear or branched, comprising 1 to 24 carbon atoms, or an aromatic group; it being understood that, when X₁ = X₂ = X₃ = Cl, k = i = 0 and n = 19, then R₁ is different from the group -(CH₂)₆-C≡C-C≡CH,
to form, on the surface of a solid support, an organised autoassembled monolayer.

11. Solid support whose surface is modified by an organised self-assembled monolayer, **characterised in that** the said monolayer comprises a lattice of at least one organosilicon compound of formula (I') as defined in Claim 10.

12. Support according to Claim 11, **characterised in that** said solid support is such that its surface has, before being modified, hydroxyl groups.

13. Support according to Claim 12, **characterised in that** the said solid support is selected from the group consisting of glasses, ceramics, metals and metalloids.

14. Method of obtaining a solid support according to any one of Claims 11 to 13, **characterised in that** it comprises the following steps:
a) eliminating contaminants from the solid support and hydrating and/or hydroxylating its surface,
b) introducing, in an inert atmosphere, at least one organosilicon compound of formula (I') as defined in Claim 10 into a mixture of at least two solvents comprising at least one non-polar hydrocarbon solvent,
c) silanisation of the support obtained at step a) by immersion in the solution prepared at step b), and
d) rinsing the modified support obtained at step c) using a solvent.

15. Method according to Claim 14, **characterised in that** step a) is performed, according to the nature of the solid support, using one or more solvents and/or oxidants and/or hydroxylants, a detergent or an ozone photochemical treatment.

16. Method according to Claim 14 or Claim 15, **characterised in that** step b) is performed in a mixture of at least one non-polar hydrocarbon solvent and at least one polar solvent.

17. Method according to Claim 16, **characterised in that** the portions by volume of the non-polar solvent and polar solvent are between 70/30 and 95/5.

18. Method according to any one of Claims 14 to 17, **characterised in that** the concentration of the organosilicon compound in the mixture of solvents, at step b) of the method, is between 1.10⁻⁵ and 1.10⁻² mole/litre.

19. Use of a solid support according to any one of Claims 11 to 13 for the synthesis or immobilisation of biomolecules by covalent bonding.

20. Method of synthesising biomolecules on a solid support according to any one of Claims 11 to 13, **characterised in that** the said biomolecules are formed by a chaining together of repetitive units and **in that** said method comprises successive steps of grafting of said repetitive units, the first repetitive unit grafted carrying a reactive function vis-à-vis the Z groups of the organosilicon compounds present on the solid support.

21. Method of immobilising biomolecules on a solid support according to any one of Claims 11 to 13, **characterised in that** it comprises a step of grafting the said biomolecules, which carry reactive functions vis-à-vis the Z groups of the organosilicon compounds, on the said solid support.

22. Method according to Claim 20 or Claim 21, **characterised in that** it is preceded, in the case where the end Z function of the organosilicon compounds is an -OCOR₁ group in which R₁ is as defined in Claim 10, or a -COOR₁ group in which R₁ is as defined in Claim 10 and is different from a hydrogen atom, by a step of deprotection of the corresponding alcohol or carboxyl acid function by an appropriate chemical treatment.
